(19) Europäisches Patentamt
European Patent Office
Office européen des brevets

(11) EP 3 229 955 B1

(12) # FASCICULE DE BREVET EUROPEEN

(45) Date de publication et mention de la délivrance du brevet:
**22.05.2019 Bulletin 2019/21**

(21) Numéro de dépôt: **15816819.5**

(22) Date de dépôt: **08.12.2015**

(51) Int Cl.:
***B01J 27/18*** *(2006.01)*
***C07C 51/377*** *(2006.01)*
***C01B 25/32*** *(2006.01)*
***C07C 69/54*** *(2006.01)*
***C07C 59/08*** *(2006.01)*
***B01J 27/138*** *(2006.01)*
***B01J 35/02*** *(2006.01)*
***C07C 67/327*** *(2006.01)*
***C07C 57/04*** *(2006.01)*

(86) Numéro de dépôt international:
**PCT/FR2015/053369**

(87) Numéro de publication internationale:
**WO 2016/092198 (16.06.2016 Gazette 2016/24)**

(54) **PRODUCTION D'ACIDES OU D'ESTERS D'ACIDES CARBOXYLIQUES INSATURÉS AVEC UN CATALYSEUR À BASE D'HALOGENO-APATITE**

VERFAHREN ZUR HERSTELLUNG VON UNGESÄTTIGTEN CARBOXYLSÄUREN ODER DESSEN ESTERN MIT EINEM HALOGENAPATIT ENTHALTENDEN KATALYSATOR

A PROCESS FOR PRODUCING UNSATURATED CARBOXYLIC ACIDS OR ESTERS THEREOF WITH A HALOGEN-APATITE BASED CATALYST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.12.2014 FR 1462186**

(43) Date de publication de la demande:
**18.10.2017 Bulletin 2017/42**

(73) Titulaires:
- **Centre National de la Recherche Scientifique 75016 Paris (FR)**
- **Ecole Centrale De Lille 59651 Villeneuve d'Ascq (FR)**
- **Université des Sciences et Technologies De Lille - Lille 1 59655 Villeneuve D'Ascq cedex (FR)**

(72) Inventeurs:
- **PAUL, Sébastien 59158 Thun St Amand (FR)**
- **KATRYNIOK, Benjamin 62410 Meurchin (FR)**
- **DUMEIGNIL, Franck 59491 Villeneuve D'ascq (FR)**
- **BONNOTTE, Thomas 75018 Paris (FR)**

(74) Mandataire: **Lavoix et al 2, place d'Estienne d'Orves 75441 Paris Cedex 09 (FR)**

(56) Documents cités:
**EP-A1- 2 495 233**

- **BLANCO E ET AL: "Gas phase dehydration of lactic acid to acrylic acid over alkaline-earth phosphates catalysts", CATALYSIS TODAY, vol. 226, 27 octobre 2013 (2013-10-27), pages 185-191, XP028627345, ISSN: 0920-5861, DOI: 10.1016/J.CATTOD.2013.09.059**
- **VIDHYA C. GHANTANI ET AL: "Catalytic dehydration of lactic acid to acrylic acid using calcium hydroxyapatite catalysts", GREEN CHEMISTRY, vol. 15, no. 5, 1 janvier 2013 (2013-01-01), page 1211, XP055206856, ISSN: 1463-9262, DOI: 10.1039/c3gc40144h**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention est relative à un procédé utilisant des halogéno-apatites comme catalyseurs de la réaction de déshydratation d'acides carboxyliques (ou d'esters) $\alpha$-hydroxylés, notamment de l'acide lactique, ainsi qu'à un procédé de production d'acides carboxyliques (ou d'esters) insaturés, notamment d'acide acrylique, en phase gaz, en présence de tels catalyseurs.

L'acide acrylique ou acide acroléïque ou acide prop-2-énoïque est un composé organique de formule brute $C_3H_4O_2$ et de formule semi-développée $CH_2{=}CHCOOH$. L'acide acrylique et ses esters, les acrylates, sont très largement utilisés dans l'industrie, notamment dans la fabrication de matières plastiques, dans les peintures acryliques et dans divers autres polyacryliques qui possèdent de multiples usages.

L'acide acrylique est généralement produit à partir du propylène, qui est un sous-produit issu des ressources fossiles, en particulier du raffinage du pétrole. En effet, la synthèse de l'acide acrylique est habituellement effectuée par oxydation en phase gaz à des températures élevées (généralement supérieures à 350°C) du propylène en deux étapes, en passant par l'acroléïne comme produit intermédiaire selon les réactions (1) et (2) suivantes :

$$CH_2{=}CH{-}CH_3 + O_2 \rightarrow CH_2{=}CH{-}CHO + H_2O \qquad (1)$$

$$CH_2{=}CH{-}CHO + ½O_2 \rightarrow CH_2{=}CH{-}COOH \qquad (2)$$

Ce procédé présente l'inconvénient de mettre en oeuvre du propylène dont le prix de revient ne cesse d'augmenter. De plus, au cours de ces transformations, divers sous-produits sont formés, provenant notamment de réactions d'oxydation partielle ou totale (acétaldéhyde, acide acétique, oxyde de carbone, dioxyde de carbone) et de polymérisation. Pour améliorer la sélectivité de la réaction, c'est-à-dire pour favoriser la production d'acroléïne et d'acide acrylique, il est nécessaire de mettre en oeuvre des catalyseurs hautement sélectifs. Il est également important de pouvoir disposer de catalyseurs performants pour pouvoir travailler à plus basses températures (<400°C).

D'autres procédés plus récents ont donc été mis au point afin de proposer des voies de synthèse de l'acide acrylique n'utilisant pas le propylène comme matière première. C'est ainsi que certains procédés proposent la préparation de l'acide acrylique par déshydratation catalytique de l'acide lactique selon la réaction (3) suivante :

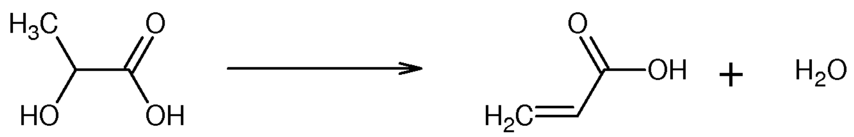

(3)

Ainsi, dans la demande de brevet EP 2 495 233, la synthèse d'acides carboxyliques insaturés tels que l'acide acrylique, est réalisée à partir d'acides hydroxycarboxyliques tels que l'acide lactique, en phase gaz en présence d'un catalyseur de type hydroxyapatite dans lequel le rapport molaire Ca/P varie de préférence de 1,5 à 8, et, en particulier, d'une hydroxyapatite de formule $Ca_{10}(PO_4)_6(OH)_2$ dans laquelle le rapport molaire Ca/P est de 1,67. Si un tel procédé permet effectivement de réaliser la synthèse d'acides carboxyliques insaturés, et en particulier de l'acide acrylique, en utilisant une matière première issue de la biomasse, il est réalisé dans un réacteur en Pyrex®, c'est-à-dire dans des conditions qui ne sont pas transposables à l'échelle industrielle où les réactions sont généralement réalisées dans des réacteurs en inox. Ainsi, les sélectivités qui sont obtenues dans les conditions de laboratoire (c-à-d, dans un réacteur en Pyrex®) se verraient diminuées de façon importante si la réaction était réalisée dans un réacteur en acier inoxydable. En effet, la demande de brevet US 2013/0274514 propose un procédé de préparation d'acide acrylique par déshydratation de l'acide lactique en présence d'un catalyseur à base d'un mélange d'hydrogénophosphates et de phosphates. Le rendement en acide acrylique dans un réacteur en quartz est de 85 % alors qu'il n'est que de 58 % lorsque la même réaction est réalisée dans des conditions opératoires identiques mais cette fois dans un réacteur en acier inoxydable. De plus, dans cette demande de brevet US 2013/0274514, une bonne reproductibilité des performances catalytiques n'est pas obtenue entre deux catalyseurs issus de deux synthèses identiques.

Il existe donc un besoin pour un procédé de préparation d'acides carboxyliques insaturés, et en particulier d'acide acrylique, de ses esters et dérivés, qui soit indépendant du propène, tout en conduisant à des rendements et sélectivités en acide acrylique qui soient acceptables lorsque la réaction est réalisée dans un réacteur en acier inoxydable de façon à être transposable à l'échelle industrielle. C'est lors de recherches à ce sujet que les présents inventeurs ont établi que des apatites ou hydroxyapatites halogénées pouvaient être utilisées pour efficacement catalyser la réaction de déshydratation d'acides carboxyliques $\alpha$-hydroxylés en phase gaz avec un très bon rendement.

La présente invention a donc pour premier objet un procédé utilisant au moins une halogéno-apatite de formule (I) suivante :

$$Ca_{10-x}[(PO_4)_{6-(x+y+z)}(HPO_4)_{x+z}(CO_3)_y][Y_{2-(x+y+z)}(CO_3)_{y+z}] \quad (I)$$

dans laquelle :

- Y représente au moins un anion choisi parmi les anions $F^-$, $Cl^-$, et les combinaisons d'au moins un anion $F^-$ ou $Cl^-$ avec un anion $OH^-$ ;
- $0 \leq x \leq 1$ ;
- $0 \leq y \leq$ ;
- $0 \leq z \leq 1$ ;
- $0 \leq x+y+z \leq 1$,

à titre de catalyseur de la réaction de déshydratation en phase gaz d'un acide carboxylique $\alpha$-hydroxylé ou d'un ester d'acide carboxylique $\alpha$-hydroxylé.

Selon une forme de réalisation préférée de l'invention, la réaction de déshydratation est réalisée sur un acide ou un ester d'acide carboxylique $\alpha$-hydroxylé de formule (II) suivante :

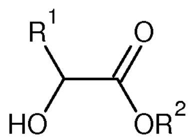

(II)

dans laquelle :

- $R^1$ est un radical alkyle possédant de 1 à 6 atomes de carbone, de préférence 1 ou 2 atomes de carbone, et de manière encore plus préférée 1 atome de carbone,
- $R^2$ représente un atome d'hydrogène ou un radical alkyle possédant de 1 à 6 atomes de carbone ; de préférence, $R^2$ représente un atome d'hydrogène, un radical méthyle ou un radical éthyle,

et conduit à l'obtention d'un acide ou d'un ester d'acide carboxylique insaturé de formule (III) suivante :

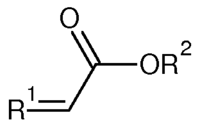

(III)

dans laquelle $R^1$ et $R^2$ ont la même signification que dans la formule (II).

Selon une forme de réalisation particulièrement préférée de l'invention, la réaction de déshydratation est réalisée à partir de l'acide lactique ($R^1$ = méthyle, $R^2$ = H) et conduit à l'obtention de l'acide acrylique.

En particulier, dans le cas de l'acide lactique, l'utilisation des halogéno-apatites de formule (I) permet d'obtenir l'acide acrylique avec un meilleur rendement (37 % environ pour l'acide acrylique) qu'en utilisant une hydroxy-apatite non halogénée (29 % environ pour l'acide acrylique), et ce, dans un réacteur en acier inoxydable, c'est-à-dire dans des conditions transposables à l'échelle industrielle.

De plus, comme évoqué précédemment, les acides carboxyliques $\alpha$-hydroxylés et leurs esters utilisés comme matière première, en particulier l'acide lactique et le lactate de méthyle, respectivement, peuvent être produits à partir de ressources renouvelables, ce qui permet de s'affranchir des ressources fossiles, et notamment du propylène, pour accéder aux acides carboxyliques $\alpha$-insaturés et à leurs esters correspondants et, en particulier, à l'acide acrylique.

Dans les halogéno-apatites de formule (I) ci-dessus, le rapport molaire Ca/P varie de préférence de 1,5 à 1,67.

Parmi les halogéno-apatites de formule (I) ci-dessus, celles dans lesquelles Y représente un anion $F^-$ ou une combinaison d'un anion $F^-$ et d'un anion $OH^-$ sont particulièrement préférées.

Selon une forme de réalisation particulièrement préférée de l'invention, la réaction de déshydratation est réalisée en présence d'une halogéno-apatite de formule (I) dans laquelle Y = F ; x = 0 ; y = 0 ; z = 0 et de rapport molaire Ca/P = 1,67.

Les surfaces spécifiques des halogéno-apatites de formule (I), (déterminées par des mesures d'adsorption de

l'azote et calcul par la méthode B.E.T.), varient généralement de 50 à 80 $m^2/g$ environ mais des solides de surfaces spécifiques inférieures et supérieures à cet intervalle peuvent tout à fait être appliqués à cette réaction.

Les halogéno-apatites de formule (I) conformes à l'invention dans lesquelles Y = un anion $F^-$ ou une combinaison d'un anion $F^-$ et d'un anion $OH^-$ peuvent être préparées par co-précipitation à pH basique, généralement compris entre 9 et 10, d'un sel de calcium, par exemple le nitrate de calcium hexahydraté et d'un phosphate, tel que, par exemple, le phosphate de diammonium, en présence de fluorure d'ammonium dans des proportions molaires correspondant, d'une part, aux valeurs de x, y et z et, d'autre part, au rapport molaire Ca/P désirés.

La réaction de précipitation des sels à pH basique est de préférence réalisée à une température variant de 65 à 80°C environ, sous agitation, et pendant une durée de 0,5 à 1 heure environ.

A la fin de la réaction de précipitation, le mélange est de préférence laissé sous agitation et chauffage pendant 5 à 6 heures.

A la fin de la synthèse, les halogéno-apatites de formule (I) peuvent être récupérées par filtration. Elles sont ensuite lavées, séchées à l'étuve, puis broyées avant d'être calcinée à une température de 380 à 430°C environ pendant 5 à 6 heures environ.

Les halogéno-apatites de formule (I) conformes à l'invention et dans lesquelles Y = un anion $Cl^-$ ou une combinaison d'un anion $Cl^-$ et d'un anion $OH^-$ peuvent être préparées par précipitation, par exemple selon la méthode décrite par S. Kannan et al, Material Letters., 2006, Volume 60(7), 864-868.

L'invention consiste en un procédé de production d'un acide carboxylique insaturé ou d'un ester d'acide carboxylique insaturé en présence d'un catalyseur, caractérisé en ce qu'il comprend au moins une étape de déshydratation d'un acide carboxylique $\alpha$-hydroxylé ou d'un ester d'un acide carboxylique $\alpha$-hydroxylé, respectivement, ladite étape étant réalisée en phase gaz, en présence d'un catalyseur solide contenant au moins une halogéno-apatite de formule (I) telle que définie ci-dessus.

Selon une forme de réalisation préférée de l'invention, l'étape de déshydratation est réalisée sur un acide ou un ester d'acide carboxylique $\alpha$-hydroxylé de formule (II) suivante :

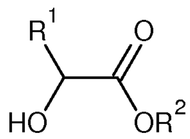
(II)

dans laquelle :

- $R^1$ est un radical alkyle possédant de 1 à 6 atomes de carbone, de préférence 1 ou 2 atomes de carbone, et de manière encore plus préférée 1 atome de carbone,

- $R^2$ représente un atome d'hydrogène ou un radical alkyle possédant de 1 à 6 atomes de carbone ; de préférence, $R^2$ représente un atome d'hydrogène, un radical méthyle ou un radical éthyle, et conduit à l'obtention d'un acide ou d'un ester d'acide carboxylique insaturé de formule (III) suivante :

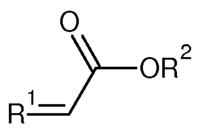
(III)

dans laquelle $R^1$ et $R^2$ ont la même signification que dans la formule (II).

Selon une forme de réalisation particulièrement préférée de l'invention, la réaction de déshydratation est réalisée sur l'acide lactique ($R^1$ = méthyle, $R^2$ = H) et conduit à l'obtention de l'acide acrylique.

Le catalyseur contient de préférence une halogéno-apatite de formule (I) dans laquelle Y = $F^-$ ; x = 0 ; y = 0 ; z = 0 et dont le rapport molaire Ca/P égal à 1,67.

La réaction de déshydratation est de préférence réalisée à une température supérieure ou égale à 300°C environ, et encore plus préférentiellement à une température variant inclusivement de 350 à 400°C environ.

Selon une forme de réalisation préférée du procédé conforme à l'invention, la réaction de déshydratation est conduite à pression atmosphérique.

De manière également préférée, la réaction de déshydratation est réalisée dans un réacteur en acier inoxydable.

Le temps de contact, défini comme étant le rapport entre le volume de catalyseur (en mL) et le débit volumique total de gaz injecté dans le réacteur (en mL/s), calculé à la température et à la pression de la réaction, varie de préférence de 0,05 à 2 s environ, et encore plus préférentiellement de 0,5 à 1,5 s environ.

De préférence, la phase gaz comprend un gaz vecteur et/ou diluant.

Le gaz vecteur et/ou diluant de la phase gaz est de préférence un gaz inerte tel que l'hélium ou l'azote.

Selon une forme de réalisation particulière du procédé conforme à l'invention, et bien que cela ne soit en aucun cas nécessaire au bon déroulement de la réaction de déshydratation, l'halogéno-apatite de formule (I) peut être supportée par un support solide préférentiellement poreux. Dans ce cas, le support solide peut, par exemple, être choisi parmi les supports à base de silice, notamment sous forme de gel de silice (type CARiACT ®), de silice mésostructurée (comme, par exemple, la silice mésostructurée de type SBA-15), ainsi que parmi les supports à base d'oxydes de silice mixtes tels que, par exemple, $SiO_2$-$TiO_2$, $SiO_2$-$ZrO_2$, voire parmi les supports de type carbure comme le carbure de silicium (SiC), etc...

Lorsqu'il est poreux, un tel support solide présente de préférence un volume poreux compris entre 0,1 $cm^3/g$ et 2,0 $cm^3/g$ inclusivement, et encore plus préférentiellement entre 0,5 $cm^3/g$ et 1,5 $cm^3/g$ inclusivement.

Lorsque la réaction est terminée, la séparation des co-produits de la réaction peut être réalisée par toutes techniques appropriées connues de l'homme de l'art, par exemple par distillation.

La présente invention est illustrée par les exemples de réalisation suivants, auxquels elle n'est cependant pas limitée.

**EXEMPLES**

Dans les exemples qui suivent, les matières premières suivantes ont été utilisées :

- Phosphate de diammonium,
- Fluorure d'ammonium,
- Nitrate de calcium,
- Ammoniaque,
- Acide lactique.

Toutes ces matières premières ont été obtenues chez Sigma Aldrich et GPR Rectapure Prolabo, et utilisées telles que reçues du fabriquant, sans purification supplémentaire.

A partir de ces matières premières, des halogéno-apatites de formule (I) avec Y = F, x = 0 et différentes teneurs en fluor ont été préparées.

La teneur en fluor dans le catalyseur final est déterminée par la quantité molaire de fluorure d'ammonium utilisée pendant la synthèse par rapport aux quantités molaires de phosphate d'ammonium et de nitrate de calcium introduites.

La synthèse de l'acide acrylique à partir de l'acide lactique a été réalisée en phase gaz dans un réacteur tubulaire en acier inoxydable à lit fixe de diamètre interne de 16 mm (diamètre externe 25 mm) et d'une longueur de 178 mm. L'injection de l'acide lactique a été réalisée à l'aide d'une pompe de chromatographie liquide haute performance (HPLC) vendue sous la dénomination commerciale 305 Pump par la société GILSON. La température du réacteur a été régulée de manière précise et contrôlée par un thermocouple de type K.

**EXEMPLE 1**

Synthèse d'une fluoro-apatite de formule $Ca_{10}(PO_4)_6F_2$ conforme à l'invention

Dans cet exemple a été réalisée la synthèse d'une halogéno-apatite de formule (I) conforme à l'invention dans laquelle Y = F, x = 0, y = 0 et z = 0 et dont le rapport molaire théorique en solution Ca/P est égal à 1,67. La formule brute du catalyseur dans ce cas est $Ca_{10}(PO_4)_6F_2$.

Une solution de phosphate de diammonium à 0,1 M dans l'eau et contenant 33,4 mmol de fluorure d'ammonium a été portée, sous agitation, à une température de 65°C dans un récipient en polytetrafluoroéthylène (PTFE). Le pH de la solution a été ajusté à 9 et maintenu à cette valeur avant et pendant la précipitation, par ajout d'une solution d'ammoniaque à 28 % en masse.

200 mL d'une solution aqueuse contenant 0,167 mol de nitrate de calcium ont ensuite été ajoutés au goutte à goutte dans la solution de phosphate de diammonium et de fluorure d'ammonium (en 1 à 2 heures) préalablement obtenue, sous agitation et à une température comprise entre 65 et 80°C. Pendant toute la durée de la précipitation, le pH du mélange a été maintenu à la valeur initiale par ajouts adaptés d'une solution d'ammoniaque à 28 % en masse.

A la fin de l'addition, le mélange a été laissé à refroidir sous agitation pendant 5 à 6 heures.

Le solide ayant précipité a ensuite été filtré sur Büchner puis lavé et filtré trois fois minimum à l'eau chaude déminéralisée. Le gâteau de solide précipité ainsi obtenu a ensuite été conservé pendant une nuit à l'étuve à 100°C.

A la sortie de l'étuve, le solide récupéré a été finement broyé puis calciné dans un four à moufle à 400°C pendant 5 à 6 heures.

Une poudre de fluoro-apatite de formule $Ca_{10}(PO_4)_6F_2$ a ainsi été obtenue.

**EXEMPLE 2**

Synthèse d'une hydroxy-fluoro-apatite de formule $Ca_{10}(PO_4)_6(OH)F$ conforme à l'invention

Dans cet exemple a été réalisée la synthèse d'une hydroxy-apatite halogénée de formule (I) conforme à l'invention dans laquelle Y représente une combinaison d'un anion $F^-$ avec un anion $OH^-$, x = 0, y = 0 et z = 0 et dont le rapport molaire théorique en solution Ca/P est égal à 1,67. La formule brute du catalyseur dans ce cas est $Ca_{10}(PO_4)_6(OH)F$.

La synthèse a été réalisée selon un mode opératoire identique à celui de l'Exemple 1 ci-dessus mais en utilisant une quantité sous-stoechiométrique de fluor, soit 16,7 mmol de fluorure d'ammonium.

Une poudre de fluoro-apatite de formule $Ca_{10}(PO_4)_6(OH)F$ a ainsi été obtenue.

**EXEMPLE 3 COMPARATIF**

Synthèse d'une hydroxyapatite non halogénée de formule $Ca_{10}(PO_4)_6(OH)_2$ non conforme à l'invention

Dans cet exemple a été réalisée la synthèse d'une hydroxyapatite non halogénée ne faisant pas partie de l'invention et ayant pour formule $Ca_{10}(PO_4)_6(OH)_2$.

La synthèse a été réalisée selon un mode opératoire identique à celui de l'Exemple 1 ci-dessus, à une température de 85°C et à pH 10, et en utilisant une solution de phosphate de diammonium ne comprenant pas de fluorure d'ammonium, tous les autres réactifs étant utilisés dans les mêmes quantités molaires que dans l'Exemple 1.

Une poudre d'hydroxyapatite de formule $Ca_{10}(PO_4)_6(OH)_2$ a ainsi été obtenue.

**EXEMPLE 4**

Synthèse de l'acide acrylique à partir de l'acide lactique

Dans cet exemple ont été testées les propriétés catalytiques d'une fluoro-apatite et d'une hydroxy-fluoro-apatite conformes à l'invention et telles que respectivement préparées selon les Exemples 1 et 2 ci-dessus, comparativement à celles de l'hydroxyapatite non halogénée non conforme à l'invention et telle que préparée ci-dessus selon l'Exemple 3 comparatif.

Le mode opératoire général suivi a été le suivant : 1 g de catalyseur broyé a été placé dans le réacteur à lit fixe en acier inoxydable et maintenu au milieu du réacteur sur un lit de laine de quartz elle-même maintenue par de la mousse métallique en acier inoxydable. Le réacteur ainsi chargé a été chauffé à la température de réaction de déshydratation de l'acide lactique, c'est-à-dire entre 350 et 375°C.

Une solution aqueuse d'acide lactique à 25% en masse a été injectée à l'aide de la pompe HPLC, à un débit de 1,5 mL/h à 6 mL/h dans une chambre d'injection chauffée à 190°C. L'évaporation de la solution d'acide lactique a été réalisée sous flux d'hélium avec un débit de 15 mL/min à 30 mL/min. L'alimentation en acide lactique a été stabilisée pendant une nuit avant le début du test catalytique.

Lors des tests catalytiques, les liquides en flux de sortie du réacteur ont été immédiatement condensés dans un piège en verre rempli d'eau, plongé dans un bain cryostaté à 4°C. Le condensat a été analysé toutes les 1h30 par chromatographie en phase gazeuse équipée d'un détecteur à ionisation de flamme.

Les calculs de conversion et de sélectivité ont été effectués de la façon suivante :

- Conversion C% = [(moles d'acide lactique injecté)–(moles d'acide lactique analysé en sortie)/(moles d'acide lactique injecté)]x100

- Sélectivité en acide acrylique S. AA % = [(moles d'acide acrylique analysé en sortie)/(moles d'acide lactique converti)]x100.

Les résultats des tests catalytiques effectués avec l'hydroxyapatite non conforme à l'invention et synthétisée selon l'Exemple 3 comparatif, avec la fluoro-apatite conforme à l'invention et telle que préparée selon l'Exemple 1 et avec l'hydroxy-fluoro-apatite conforme à l'invention et telle que préparée selon l'Exemple 2 sont présentés dans les Tableaux I et II ci-après :

**TABLEAU I**

| Catalyseur | C % | S. AA % | Rendement % |
|---|---|---|---|
| $Ca_{10}(PO_4)_6(OH)_2$ (Ex. 3) | 97 | 30 | 29,1 |
| $Ca_{10}(PO_4)_6F_2$ (Ex. 1) | 100 | 37 | 37,0 |
| Température de réaction : 350°C ; solution d'acide lactique à 25% en masse ; débit de la solution d'acide lactique : 1,5 mL/h, débit d'hélium : 15 mL/min. | | | |

**TABLEAU II**

| Catalyseur | C % | S. AA % | Rendement % |
|---|---|---|---|
| $Ca_{10}(PO_4)_6(OH)_2$ (Ex. 3) | 93,4 | 34,3 | 32,0 |
| $Ca_{10}(PO_4)_6(OH)F$ (Ex. 2) | 93,3 | 36,1 | 33,7 |
| $Ca_{10}(PO_4)_6F_2$ (Ex. 1) | 99 | 38,5 | 38,1 |
| Température de réaction : 375°C ; solution d'acide lactique à 25% en masse ; débit de la solution d'acide lactique : 6 mL/h, débit d'hélium : 30 mL/min. | | | |

Les résultats des Tableaux I et II montrent que les apatites et hydroxyapatites contenant du fluor, et conformes à la présente invention, permettent d'obtenir de meilleurs rendements en acide acrylique que l'hydroxy-apatite de référence non halogénée, dans des conditions opératoires données.

L'ensemble des résultats présentés dans cette invention, dans diverses conditions, met en évidence que des halogéno-apatites testées pour la déshydratation de l'acide lactique en acide acrylique sont performantes et sélectives en acide acrylique, et ce, dans des conditions transposables à l'échelle industrielle.

## Revendications

1. Procédé de production d'un acide carboxylique insaturé ou d'un ester d'acide carboxylique insaturé en présence d'un catalyseur, **caractérisé en ce qu'**il comprend au moins une étape de déshydratation d'un acide carboxylique $\alpha$-hydroxylé ou d'un ester d'un acide carboxylique $\alpha$-hydroxylé, respectivement, ladite étape étant réalisée en phase gaz, en présence d'un catalyseur solide contenant au moins une apatite halogénée de formule (I) suivante :

$$Ca_{10-x}(PO_4)_{6-(x+y+z)}(HPO_4)_{x+z}(CO_3)_y][Y_{2-(x+y+z)}(CO_3)_{y+z}] \qquad (I)$$

dans laquelle :

- Y représente au moins un anion choisi parmi les anions $F^-$, $Cl^-$ et les combinaisons d'au moins un anion $F^-$ ou $Cl^-$ avec un anion $OH^-$ ;
- $0 \leq x \leq 1$ ;
- $0 \leq y \leq 1$ ;
- $0 \leq z \leq 1$ ;
- $0 \leq x+y+z \leq 1$.

2. Procédé selon la revendication 1, **caractérisé en ce que** l'étape de déshydratation est réalisée sur un acide ou un ester d'acide carboxylique $\alpha$-hydroxylé de formule (II) suivante :

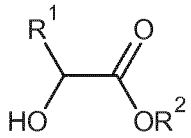 (II)

dans laquelle :

- $R^1$ est un radical alkyle possédant de 1 à 6 atomes de carbone,
- $R^2$ représente un atome d'hydrogène ou un radical alkyle possédant de 1 à 6 atomes de carbone ;

et conduit à l'obtention d'un acide ou d'un ester d'acide carboxylique insaturé de formule (III) suivante :

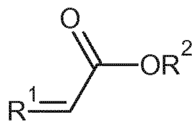 (III)

dans laquelle $R^1$ et $R^2$ ont la même signification que dans la formule (II).

3. Procédé selon la revendication 2, **caractérisé en ce que** l'étape de déshydratation est réalisée sur un acide ou un ester d'acide carboxylique $\alpha$-hydroxylé de formule (II) dans laquelle $R^1$ est un radical alkyle possédant 1 atome de carbone, et $R^2$ représente un atome d'hydrogène, un radical méthyle ou un radical éthyle.

4. Procédé selon l'une des revendication 1 à 3, **caractérisé en ce que** l'étape de déshydratation est réalisée à partir de l'acide lactique et conduit à l'obtention de l'acide acrylique.

5. Procédé selon l'une quelconque des revendications 1 à 4, **caractérisé en ce que** le catalyseur contient au moins une halogéno-apatite de formule (I) dans laquelle $Y = F^-$ ; x = 0 ; y = 0 ; z = 0 et de rapport molaire Ca/P = 1,67.

6. Procédé selon l'une quelconque des revendications 1 à 5, **caractérisé en ce que** la réaction de déshydratation est réalisée à une température supérieure ou égale à 300°C.

7. Procédé selon l'une quelconque des revendications 1 à 6, **caractérisé en ce que** la réaction de déshydratation est conduite à pression atmosphérique.

8. Procédé selon l'une quelconque des revendications 1 à 7, **caractérisé en ce que** la réaction de déshydratation est réalisée dans un réacteur en acier inoxydable.

9. Procédé selon l'une quelconque des revendications 1 à 8, **caractérisé en ce que** la phase gaz comprend un gaz vecteur et/ou diluant.

10. Procédé selon la revendication 9, **caractérisé en ce que** le gaz vecteur et/ou diluant de la phase gaz est un gaz inerte choisi parmi l'hélium et l'azote.

11. Procédé selon l'une quelconque des revendications 1 à 10, **caractérisé en ce que** l'halogéno-apatite de formule (I) est supportée par un support solide.

**Patentansprüche**

1. Verfahren zur Herstellung einer ungesättigten Carbonsäure oder eines Esters einer ungesättigten Carbonsäure in Anwesenheit eines Katalysators, **gekennzeichnet dadurch, dass** es mindestens einen Schritt der Dehydratisierung einer $\alpha$-Hydroxycarbonsäure beziehungsweise eines Esters einer $\alpha$-Hydroxycarbonsäure umfasst, wobei der Schritt in der Gasphase durchgeführt wird, in Anwesenheit eines festen Katalysators enthaltend mindestens ein halogeniertes Apatit der folgenden Formel (I):

$$Ca_{10-x}[PO_4)_{6-(x+y+z)}(HPO_4)_{x+z}(CO_3)_y][Y_{2-(x+y+z)}(CO_3)_{y+z}] \quad (I)$$

wobei:

- Y mindestens ein Anion ausgewählt aus den Anionen $F^-$, $Cl^-$ und Kombinationen mindestens eines Anions $F^-$ oder $Cl^-$ mit einem Anion $OH^-$ darstellt;
- $0 \leq x \leq 1$;
- $0 \leq y \leq 1$;
- $0 \leq z \leq 1$;
- $0 \leq x+y+z \leq 1$.

2. Verfahren gemäß Anspruch 1, **gekennzeichnet dadurch, dass** der Schritt der Dehydratisierung an einer α-Hydroxycarbonsäure oder einem Ester davon der folgenden Formel (II) durchgeführt wird:

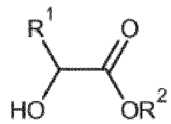 (II)

wobei:

- $R^1$ ein Alkylradikal mit von 1 bis 6 Kohlenstoffatomen ist,
- $R^2$ ein Wasserstoffatom oder ein Alkylradikal mit von 1 bis 6 Kohlenstoffatomen darstellt; und zum Erhalt einer ungesättigten Carbonsäure oder eines Esters davon der folgenden Formel (III) führt:

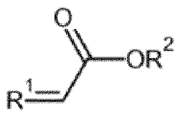 (III)

wobei $R^1$ und $R^2$ die gleiche Bedeutung wie in Formel (II) haben.

3. Verfahren gemäß Anspruch 2, **gekennzeichnet dadurch, dass** der Schritt der Dehydratisierung an einer α-Hydroxycarbonsäure oder einem Ester davon der Formel (II) durchgeführt wird, wobei $R^1$ ein Alkylradikal mit 1 Kohlenstoffatom ist und $R^2$ ein Wasserstoffatom, ein Methylradikal oder ein Ethylradikal darstellt.

4. Verfahren gemäß einem der Ansprüche 1 bis 3, **gekennzeichnet dadurch, dass** der Schritt der Dehydratisierung ausgehend von Milchsäure erfolgt und zum Erhalt von Acrylsäure führt.

5. Verfahren gemäß einem der Ansprüche 1 bis 4, **gekennzeichnet dadurch, dass** der Katalysator mindestens ein Halogenapatit der Formel (I) enthält, wobei $Y = F^-$; $x = 0$; $y = 0$; $z = 0$ und das Molverhätlnis Ca/P = 1,67.

6. Verfahren gemäß einem der Ansprüche 1 bis 5, **gekennzeichnet dadurch, dass** die Dehydratisierungsreaktion bei einer Temperatur höher als oder gleich 300 °C durchgeführt wird.

7. Verfahren gemäß einem der Ansprüche 1 bis 6, **gekennzeichnet dadurch, dass** die Dehydratisierungsreaktion bei atmosphärischem Druck durchgeführt wird.

8. Verfahren gemäß einem der Ansprüche 1 bis 7, **gekennzeichnet dadurch, dass** die Dehydratisierungsreaktion in einem Edelstahlreaktor durchgeführt wird.

9. Verfahren gemäß einem der Ansprüche 1 bis 8, **gekennzeichnet dadurch, dass** die Gasphase ein Träger- und/oder ein Verdünnungsgas umfasst.

10. Verfahren gemäß Anspruch 9, **gekennzeichnet dadurch, dass** das Träger- und/oder Verdünnungsgas der Gasphase ein inertes Gas ausgewählt aus Helium und Stickstoff ist.

**11.** Verfahren gemäß einem der Ansprüche 1 bis 10, **gekennzeichnet dadurch, dass** das Halogenapatit der Formel (I) von einem festen Träger getragen wird.

## Claims

**1.** Process for producing an unsaturated carboxylic acid or an unsaturated carboxylic acid ester in the presence of a catalyst, **characterized in that** it comprises at least one step of dehydrating an $\alpha$-hydroxylated carboxylic acid or an $\alpha$-hydroxylated carboxylic acid ester, respectively, said step being carried out in the gas phase, in the presence of a solid catalyst containing at least one halogenated apatite of formula (I) below:

$$Ca_{10-x}[(PO_4)_{6-(x+y+z)}(HPO_4)_{x+z}(CO_3)_y][Y_{2-(x+y+z)}(CO_3)_{y+z}] \qquad (I)$$

in which:

- Y represents at least one anion selected from the $F^-$, $Cl^-$ anions, and the combinations of at least one $F^-$ or $Cl^-$ anion with an $OH^-$ anion;
- $0 \leq x \leq 1$;
- $0 \leq y \leq 1$;
- $0 \leq z \leq 1$;
- $0 \leq x+y+z \leq 1$.

**2.** Process according to claim 1, **characterized in that** the dehydration step is carried out on an $\alpha$-hydroxylated carboxylic acid or acid ester of formula (II) below:

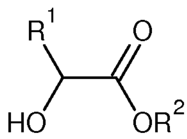 (II)

in which:

- $R^1$ is an alkyl radical having from 1 to 6 carbon atoms,
- $R^2$ represents a hydrogen atom or an alkyl radical having from 1 to 6 carbon atoms;

and results in an unsaturated carboxylic acid or acid ester of formula (III) below being obtained:

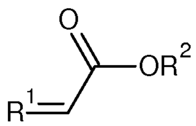 (III)

in which $R^1$ and $R^2$ have the same meaning as in formula (II).

**3.** Process according to claim 2, **characterized in that** the dehydration step is carried out on an $\alpha$-hydroxylated carboxylic acid or acid ester of formula (II) in which $R^1$ is an alkyl radical having 1 carbon atom, and $R^2$ represents a hydrogen atom, a methyl radical or an ethyl radical.

**4.** Process according to any one of claims 1 to 3, **characterized in that** the dehydration step is carried out using lactic acid and results in acrylic acid being obtained.

**5.** Process according to any one of claims 1 to 4, **characterized in that** the catalyst contains at least one haloapatite of formula (I) in which $Y = F^-$; x = 0; y = 0; z = 0 and of Ca/P molar ratio = 1.67.

**6.** Process according to any one of claims 1 to 5, **characterized in that** the dehydration reaction is carried out at a temperature greater than or equal to 300°C.

7. Process according to any one of claims 1 to 6, **characterized in that** the dehydration reaction is carried out at atmospheric pressure.

8. Process according to any one of claims 1 to 7, **characterized in that** the dehydration reaction is carried out in a stainless steel reactor.

9. Process according to any one of claims to 1 to 8, **characterized in that** the gas phase comprises a carrier gas and/or diluent gas.

10. Process according to claim 9, **characterized in that** the carrier gas and/or diluent gas of the gas phase is an inert gas selected from helium and nitrogen.

11. Process according to any one of claims 1 to 10, **characterized in that** the haloapatite of formula (I) is supported by a solid support.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2495233 A
- US 20130274514 A


### Littérature non-brevet citée dans la description


- **S. KANNAN et al.** *Material Letters.,* 2006, vol. 60 (7), 864-868